# EUROPEAN PATENT SPECIFICATION

(11) **EP 0 370 090 B1**
(45) Date of publication and mention of the grant of the patent: **10.07.1996**
(21) Application number: 89905259.1
(22) Date of filing: 28.04.1989
(51) Int. Cl.: C07K 1/00, C07K 2/00, C07K 7/00, C12P 21/00, A61K 38/00

(54) **IMMUNOGENS AND BIOLOGICALLY ACTIVE PEPTIDES DERIVED FROM SHARED SEQUENCES FROM ANTIGENS AND ANTI-IDIOTYPIC ANTIBODIES OR ANTIBODIES SPECIFIC FOR CELLULAR RECEPTORS OF THE ANTIGENS**
VON GEMEINSAMEN SEQUENZEN VON ANTIGENEN UND ANTIIDIOTYPISCHEN ANTIKÖRPERN ODER VON ANTIKÖRPERN MIT SPEZIFIZITÄT FÜR DIE ZELLULÄREN REZEPTOREN DER ANTIGENE ABGELEITETE IMMUNOGENE UND BIOLOGISCH AKTIVE PEPTIDE
IMMUNOGENES ET PEPTIDES BIOLOGIQUEMENT ACTIFS DERIVES DE SEQUENCES PARTAGEES D'ANTIGENES ET D'ANTICORPS ANTI-IDIOTYPIQUES OU D'ANTICORPS SPECIFIQUES CONTRE DES RECEPTEURS CELLULAIRES DES ANTIGENES

(30) Priority: 13.05.1988 US 194026; 21.03.1989 US 326328
(43) Date of publication of application: 30.05.1990
(73) Proprietor: UNIVERSITY PATENTS, INC., Westport, Connecticut 06881 (US)
(72) Inventor: GREENE, Mark, I., Penn Valley, PA 19072 (US); WILLIAMS, William, V., Havertown, PA 19083 (US); WEINER, David, B., Penn Wynn, PA 19151 (US); COHEN, Jeffrey, Bala Cynwyd, PA 19004 (US)
(74) Representative: Stebbing, Peter John Hunter
(86) International application number: US8901815
(87) International publication number: WO8910939

(56) References cited:
- EP-A- 0 241 139
- WO-A-87/02990
- WO-A-88/07375
- WO-A-88/09181
- US-A- 4 683 295
- US-A- 4 761 371
- TECHNOLOGICAL ADVANCES IN VACCINE DEVELOPMENT, SYMPOSIA ON MOLECULAR AND CELLULAR BIOLOGY, NEW SERIES, vol. 84, TECHNOLOGICAL ADVANCES IN VACCINE DEVELOPMENT; GENENTECH, ORTHO, SMITH KLINE AND FRENCH-UCLA SYMPOSIUM, Utah, 30th January - 6th February 1988, pages 577-586, Alan R. Liss, Inc.; W.V. WILLIAMS et al.: "Determination of the neutralizing/cell-attachment epitope of reoviurs type 3"
- ANN. INST. PASTEUR IMMUNOL., vol. 136 D, 1985, pages 259-269, Elsevier, Paris, FR; G. MAZZA et al.: "A structural basis for the internal image in the idiotypic network: antibodies against synthetic Ab2-D regions cross-react with the original antigen"
- THE JOURNAL OF IMMUNOLOGY, vol. 138, no. 1, 1st January 1987, pages 7-9, The American Association of Immunologists, US; L R Smith et al.
- PROC. NATL. ACAD. SCI. USA, vol. 85, September 1988, pages 6488-6492; W.V. WILLIAMS et al.: "Sequences of the cell-attachment sites of reovirus type 3 and its anti-idiotypic/antireceptor antibody: modeling of their three-dimensional structures"
- Journal of Immunology Vol. 128, pp. 247-250. Jan 1982. R. B. FRITZ et al. "Idiotypes of Lewis Rat Antibodies. . ." see Abstract and p. 250.
- Journal of Immunology Vol. 136, pp. 582-7 Jan 1986 M. V. SEIDEN, "Hypervariable Region Peptides Variably Induce Specific Anti-Idiotype Antibodies. . ." see: Abstract and p. 586.
- Journal of Immunology Vol. 140, pp. 3059-65 May 1988 M. BUDISAVLJEVIC, et al. "Angiotensin II (AII)-Related Idiotypic Network. see: Abstract and p. 3064.
- R. A. LERNER, et al. (Editors), Vaccines 85, Cold Spring Harbor Laboratory, New York 11724, published 1985. See page 151-156
- Proceedings of the National Academy of Sciences, Vol. 84, pp. 3891-3895. June 1987, T. C. CHANH, et al. "Monoclonal Anti-Idiotypic Antibody Mimics the CD4 Receptor and Binds Human Immunodeficiency Virus." see: Abstract and p. 3895.
- Proceedings of the National Academy of Sciences, Vol. 83, pp. 6578-6582. Sep 1986, C. BRUCK, et al. "Nucleic Acid Sequence of an Internal Image-Bearing Monoclonal Anti-Idiotype and its Comparison to he Sequence of the External Antigen. see: Abstract and p. 6582.
- Journal of Experimental Medicine, Vol 164, pp. 227-236. July 1986. Y.M. THANAVAL, et al. "A Surrogate Hepatitis B Virus Antigenic Epitope Represented by a Syntetic Peptide and an Internal Image Anti-Idiotype Antibody. see: Summary and p. 235.
- Cancer Research, Vol. 45, pp. 6119-6123, Dec 1985, L. J. SMITH, et al. "Production of Heterologous Antibodies for Murine B-Cell Leukemia Immunoglobulin by Immunization with Synthetic Peptides Homologous to Heavy Chain Hypervariable Regions. see: Abstract and pp. 6122-3.
- Molecular and Cellular Biochemistry, Vol 65, pp. 5-21, Nov 1984, C.N. GAULTON, et al. "Anti-Idiotypic Antibodies as Probes of Cell Surface Receptors." see: Abstract as Cited in Dialog Database File 155, Acc. Number 85110880.
- R. ARNON (Editor), Synthetic Vaccines, Vol. 1, 1987, CRC Press, Boca Raton Florida. see Abstract as Cited in Dailog Database File 5, Biosis No. 35080391.

## Description

The present invention relates to the field of agents which are useful in the immunization of mammals against infectious organisms, such as bacteria, fungi, parasites, and viruses and neoplasms which express specific antigens. Such organisms typically have specific sites which bind to a complementary portion, called a receptor site, of the host cells. This invention also relates to the field of biologically active agents, such as pharmaceuticals, which act on mammalian cells by binding to the receptor site(s) of those cells to alter or affect their function or behaviour, or to prevent or alter the effect which another agent, such as an antigen or pathogen, would otherwise have upon those cells.

Several strategies have been employed to develop safe, effective vaccines against viral and bacterial pathogens. At present most vaccines in use consist of live attenuated pathogens, inactivated pathogens, components of a pathogen, or modified toxins (toxoids). See Institute of Medicine, "Vaccine Supply and Innovation", Washington, D.C.: National Academy Press (1985). While these preparations have been successfully used for many infectious diseases, many pathogens exist where these approaches have not worked or have not been applicable. Certain pathogens are potentially too dangerous to contemplate the use of attenuated or even inactivated preparations. The risk of developing cancer from immunization with certain retroviruses, or of developing acquired immunodeficiency syndrome (AIDS) from immunization with human immunodeficiency virus (HIV) underscores the drawbacks associated with the use of whole virus preparations for vaccination. In addition many pathogens display a marked antigenic heterogeneity that makes effective vaccination difficult. These considerations have led us to seek alternative method for effective immunization.

The idiotype network theory of N.K. Jerne, "Towards a Network Theory of the Immune system", Ann. Immunol. (Paris) 125: 337-389, (1974) implies that an anti-idiotypic antibody raised against a neutralizing antibody specific for a pathogen would mimic that pathogen immunologically. Immunization with the anti-idiotype should result in the development of a significant anti-pathogen response with the elicitation of neutralizing antibodies and cell-mediated immunity. In recent years there have been several examples where this strategy has been effective, including reovirus type 3. See Sharpe, A.H., Gaulton, G.N., McDade, K.K., Fields, B.N., and Greene, M.I., "Syngeneic Monoclonal Antiidiotype Can Induce Cellular Immunity to Reovirus", J. Exp. Med., 160: 195-205 (1984); Kauffman, R.S., Noseworthy, J.H., Nepom, J.T., Finberg, R., Fields, B.N., and Greene, M.I., "Cell Receptors for Mammalian Reovirus II: Monoclonal Anti-Idiotypic Antibody Blocks Viral Binding to Cells", J. Immunol. 131:2539-2541, (1983): and Gaulton, G.N., Sharpe, A.H., Chand, D.W., Fileds, B.N. and Greene, M.I. (1986). "Syngeneic monoclonal internal image anti-idiotopes as prophylactic vaccines". J. Immunol. 137: 2930-2936. With respect to Sendai virus see Ertl, H.C. and Finberg, R.W., "Sendai Virus Specific T Cell Clones: Induction of Cytolytic T Cells by an Anti-Idiotypic Antibody Directed Against a Helper T Cell Clone", Proc. Natl. Acad. Sci. USA. 81:2850-2854, (1984). For report relating to rabies see Reagen, K.J., Wanner, W.H., Wiktor, T., and Koprowski, H., "Anti-Idiotypic Antibodies Induce Neutralizing Antibodies to Rabies Virus Glycoproteins", J. Virol., 48:660-666, (1983). This approach has been discussed in connection with polio virus in Uydeltaag, F.G.C.M. and Osterhaus, A.D.M.E., "Induction of Neutralizing Antibody in Mice Against Polio Virus Type II with Monoclonal Antiidiotypic Antibody", J. Immunol., 134:1225-1229, (1985). One of the key aspects of this approach is that the anti-idiotype mimics a neutralizing epitope on the pathogen and induces a neutralizing response. Thus a potent anti-idiotype vaccine would seem to be an ideal immunogen in cases where intact pathogen could not be used or where irrelevant non-neutralizing epitopes dominate the immune response. However, the practical application of anti-idiotypes as vaccine has been limited by the difficulties in making human monoclonal antibodies and in the danger of producing serum sickness by using xenogeneic antibodies.

Another method currently under intensive investigation is the use of synthetic peptides corresponding to segments of the proteins from pathogenic microorganisms against which an immune response is directed. This approach has been successful in several instances including feline leukemia virus (Elder, J.H., McGee, J.S., Munson, N.M., Houghten, R.A., Xloetzer, W., Bittle, J. L., and Grant, C.K.," Localization of Neutralizing Regions of the Envelope Gene of Feline Leukemia Virus by Using Anti-Synthetic Peptide Antibodies", J. Virol., 61:8-15, 1987), hepatitis B (Gerin, J.L., Alexander, H., Shih, J.W., Purcell, R.H., Dapolito, G., Engle, R., Green, N., Sutcliffe, J.G., Shinnick, T.M., and Lerner, R.A., "Chemically Synthesized Peptides of Hepatitis B Surface Antigen Duplicate the D/Y Specificities and Induce subtype-SpecificAntibodies in Chimpanzees", Proc. Natl. Acad. Sci. USA. 80:2365-2369, 1983), Plasmodium falciparum (Cheung, A., Leban, J., Shaw, A.R., Merkli, B., Stocker, J., Chizzolini, C., Sander, C., and Perrin, L.H., "Immunization With Synthetic Peptides of a Plasmodium Fulciparum Surface Antigen Induces Antimerozoite Antibodies", Proc. Natl. Acad. Sci. USA*,* 83: 8328-8332, 1986), cholera toxin (Jacob, C.O., Grossfeld, S., Sela, M., and Arnon, R., "Priming Immune Response to Cholera Toxin Induced by Synthetic Peptides", Eur. J. Immunol., 16:1057-1062, 1986) and others. When these peptides are capable of eliciting a neutralizing immune response they appear to be ideal immunogens. They elicit a specific response and typically do not lead to deleterious effects on the host. However, it can be difficult to predict which peptide fragments will be immunogenic and lead to the development of a neutralizing response. It would be desirable to develop immunogens that elicit a response to specific neutralizing epitopes without causing responses to extraneous epitopes that could "dilute" the specific response or lead to harmful immune complex formation.

Technological advances in vaccine development; symposia on molecular biology, Williams W V et al 1988 Vol 84 pp 577-588 discloses individual peptides V_{L} and Vₕ and show sequence homology with Reo peptides. Similarly individual sequence homology is revealed between relevant peptides and target sequences in Ann Inst Pasteur. Immunol. Vol 136D 1985 pp 259-269 and in Proc. Natl. Acad. Sci. USA. Bruck C. et al 1986 Vol. 83 pp 6578-6582.

According to the present invention therefore there is provided a method of synthesising biologically active compounds comprising the steps of:
(a) selecting a pathogen product known to bind to a physiological receptor of a mammalian cells (antigen);
(b) providing an antibody for a cellular receptor of said pathogen gene product;
(c) Determining and comparing the sequences of antigen and the V_{L} and V_{H} regions of the determining region of said antibodies;
(d) synthesizing a peptide comprising an epitope whose sequence matches a homologous region determined as in (c) having at least 60% sequence homology between the V_{L} and V_{H} sequence and the sequence of the antigen,
characterised by the further steps of (e) adding cysteine to one end of a peptide of step (d),
(f) adding cysteine to the one end of another peptide of step (d); and
(g) contacting said peptides under conditions selected to allow binding at said cysteine on each peptide to form a composite peptide dimer.

The present invention thus provides a novel method for determining the desirable amino acid sequences of biologically active peptides to thereby provide an immunogen capable of eliciting a response to specific neutralizing epitopes without causing response to extraneous epitopes that could "dilute" the desired response or lead to harmful immune complex formation. The invention is predicated upon the surprising discovery that antibodies specific for a cellular receptor of an antigen exhibit a peptide sequence homology to the peptide sequence found in the corresponding region of the antigen itself. Accordingly, instead of using anti-receptor antibody itself as the vaccine or the like, it is used only to identify the shared peptide sequences between corresponding regions of the antigen and anti-receptor antibody. A peptide comprising that shared sequence is then synthesized for therapeutic use.

In the preferred embodiment, the antigen is a virus such as a reovirus, and the peptide comprises at least sixty, preferably eighty to one hundred percent sequence homology found in an antigen region and the corresponding region of the anti-receptor antibody.

The present invention is useful to provide a novel vaccine for immunizing a host mammal against an infectious organism which has a site which binds specifically to a receptor site on a host cell. The vaccine may be utilized by the step of inoculating the mammal with a synthetic biologically active peptide having a peptide sequence corresponding to a peptide sequence found in both said site of said infectious organism and in an antibody specific for a receptor on a host cell, in an amount effective to reduce the likelihood that said host will be susceptible to infection by said organism. This is particularly suited to immunizing a host mammal against infections caused by a virus, such as a reovirus.

Demonstration of the feasibility and utility of the products of the present invention has been accomplished using reovirus 3, a virus which is known to specifically bind to a structure physically similar to the beta-adrenergic receptor of mammalian cells to alter the metabolic state of those cells.

The thus synthesized peptide at least comprises, and preferably consists essentially of, at least six amino acids of the shared sequence. The gene products selected in accordance with step (a) include reovirus gene products, such as those of reovirus 3; vaccinis gene products; Epstein-Barr virus gene products; and gene products which bind to the mammalian beta-adrenergic receptor family.

Novel in vitro methods of altering the growth of mammalian cells are also provided by the present invention. These methods include the step of administering to mammalian cells a peptide comprising a sequence of amino acids which is shared by a pathogen gene product known to bind to the beta-adrenergic receptor of that cell and by an antibody specific for said beta-adrenergic receptor, said sequence being selected and said peptide being administered in an amount effective to alter the growth of said cells. The preferred peptides and pathogen gene products for use in these methods are those as described above.

Accordingly, a primary object of the present invention is the provision of novel, biologically active peptides.

Another object of the present invention is the provision of novel methods for producing such peptides which exhibit biologic activity.

These and other objects of the present invention will become apparent from the following, more detailed description.

### BRIEF SUMMARY OF THE FIGURES

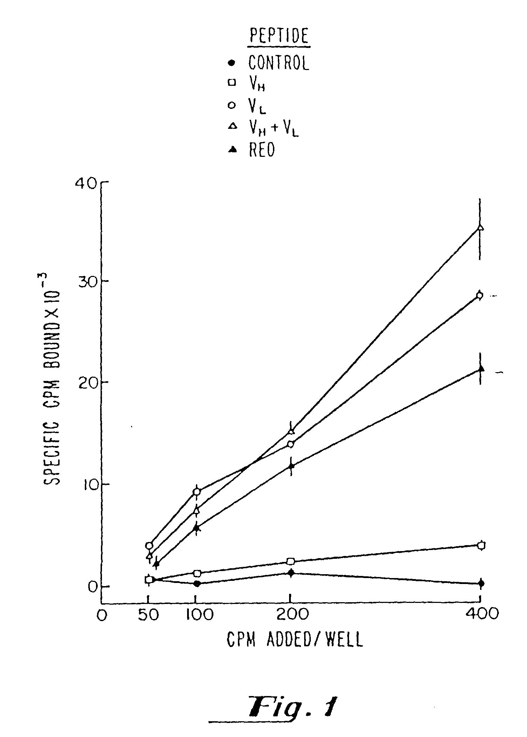

Figure 1 illustrates the specific binding of 9BG5 to peptides, determined by radioimmunoassay as noted in the experimental procedures described hereinafter; CPM of 9BG5 bound to blank wells was subtracted from CPm of 9BG5 bound to peptide coated wells; non-specific binding to peptides was corrected for by subtracting from the value a similar value determined for an isotype-matched control monoclonal antibody UPC10; specific CPM of 9BG5 bound to peptide coated wells is shown using the amount of 9BG5 added to each well in a final volume of 50 ul.; mean + SD for duplicate wells is shown.

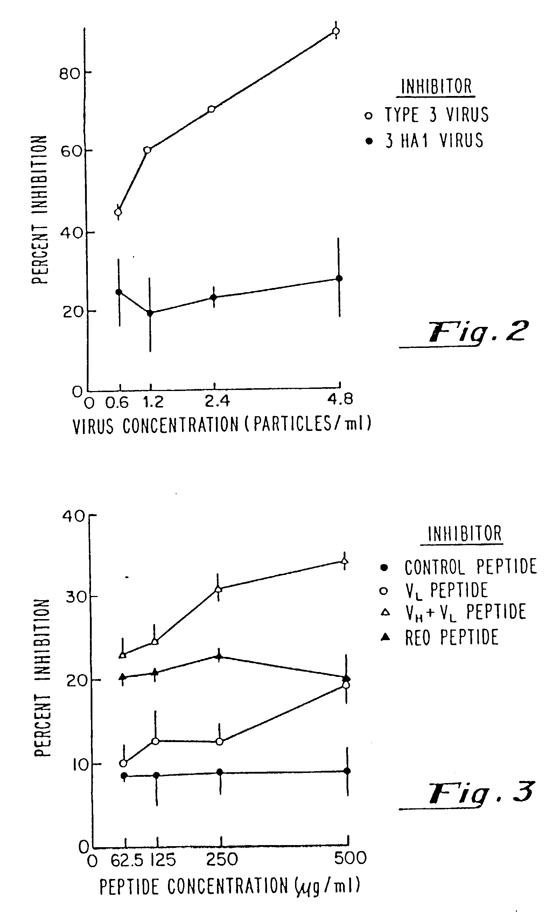

Figures 2 and 3 illustrate the binding of V_{L}-BSA to type 3 reovirus receptor as determined by its ability to compete for binding with anti-reovirus type 3 receptor antibody 87.92.6.; R1.1 cells (10⁷/ml) were incubated in 1% BSA in the presence or absence of 200 ug/ml V_{L}-BSA or V_{H}-BSA as indicated for 45 minutes; monoclonal antibodies were added at the concentrations noted for an additional 30 minutes; the cells were washed twice and a 1:200 dilution of FITC-goat anti-mouse Fab was added for 30 minutes; the cells were washed twice and analyzed for fluorescence intensity on a FACS analyzer; percent maximal cell staining was determined as the ratio of the percent of the cells positive on FACS analysis at the antibody concentration noted to the maximal percent of cells judged positive at saturating doses of monoclonal antibody in the absence of competitors ([% positive at concentration divided by maximal % positive] X 100); the maximal percent positive values were as follows: 2 - 15.3%, 3 - 97%, 3 - 24%.

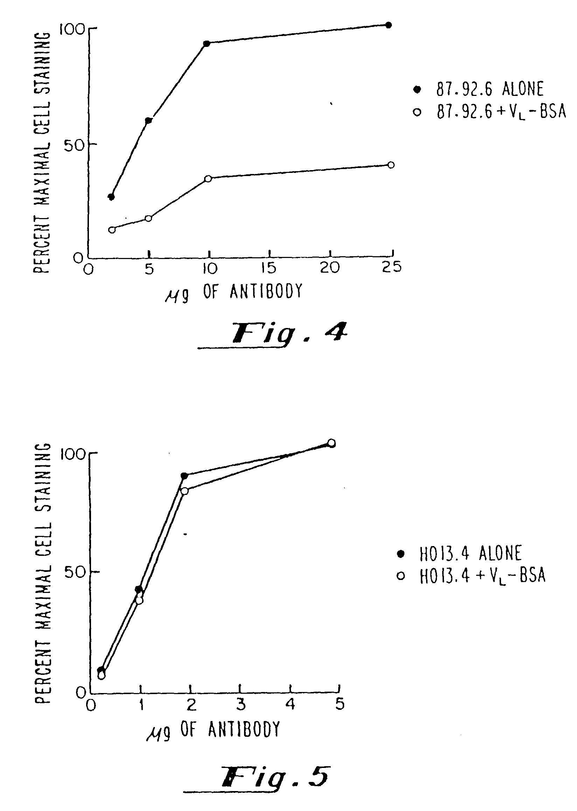

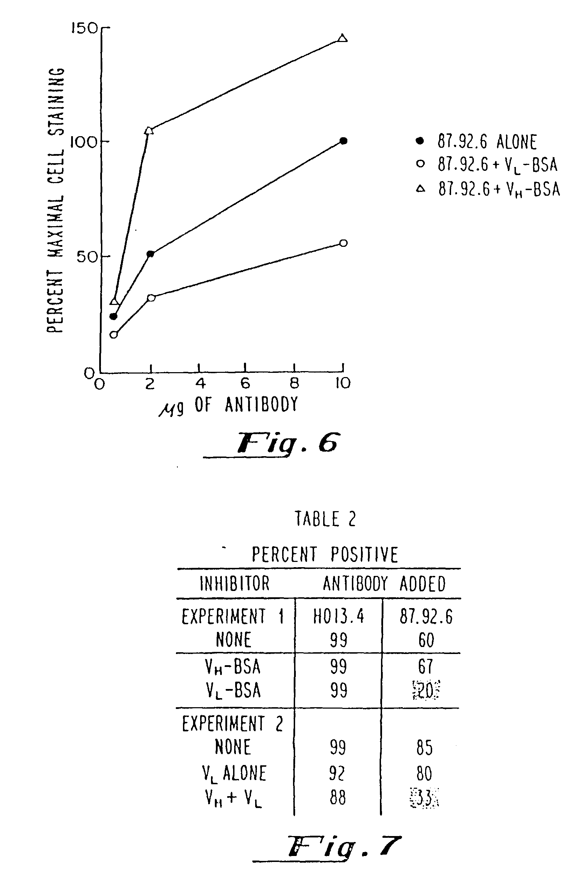

Figures 4-7 illustrate specific binding of immune serum to virus-coated plates, determined by radioimmunoassay as noted in the hereinafter described experimental procedures; CPM of immune serum binding to blank wells was subtracted from CPM binding to virus coated wells; to account for non-specific binding to virus coated wells, a similar value determined for normal mouse serum was subtracted form the value determined for immune serum; specific CPM bound is shown versus the dilution of mouse serum added in a final volume of 50 ul.; the mean ± SEM of duplicate wells from groups of 3 or 4 mice is shown at each dilution.

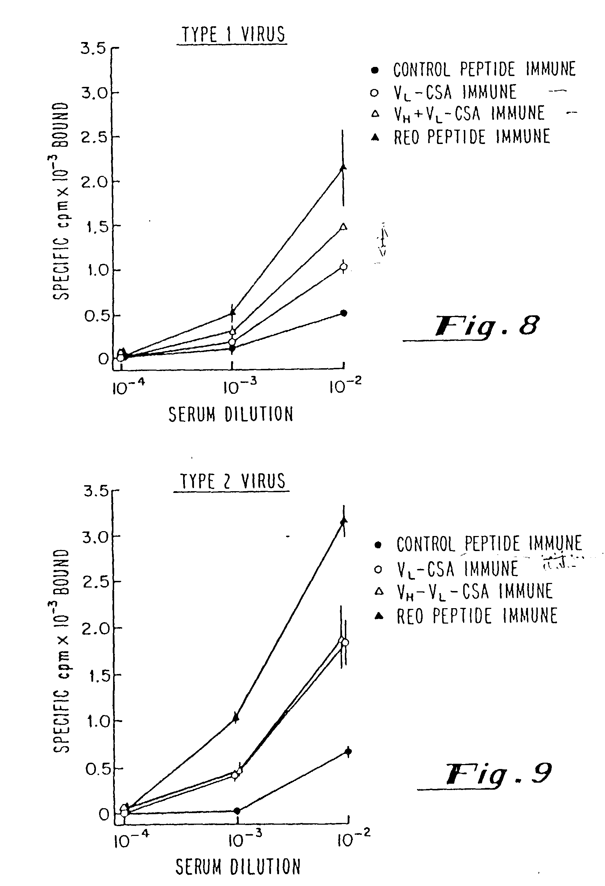

Figures 8 and 9 illustrate immune serum assays for viral neutralization as described in the following section; serum was collected prior to immunization with peptides (pre-immune or day 0), on day 20 following the first immunization, and on day 60; the neutralization titer was determined at each time point from groups of 4 mice; the geometric mean divided by SEM of the reciprocal of the neutralization titer is shown at each time point.

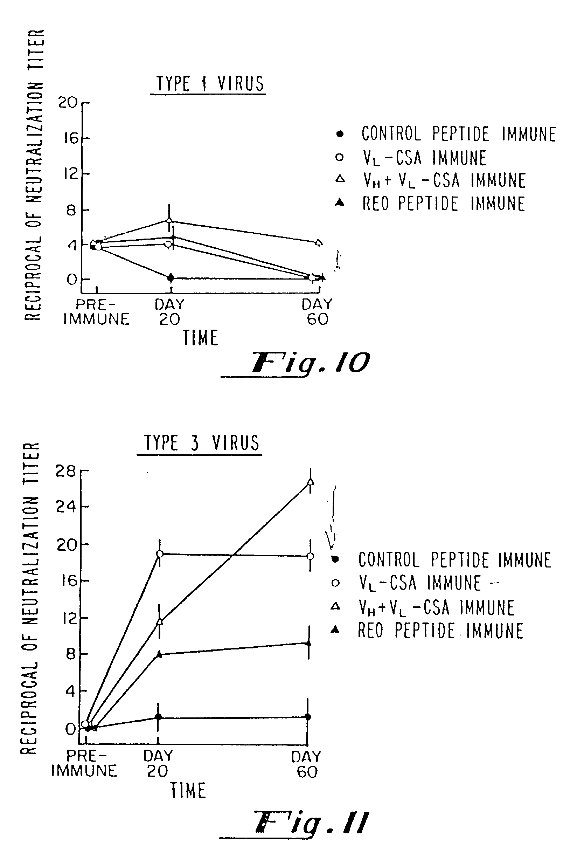

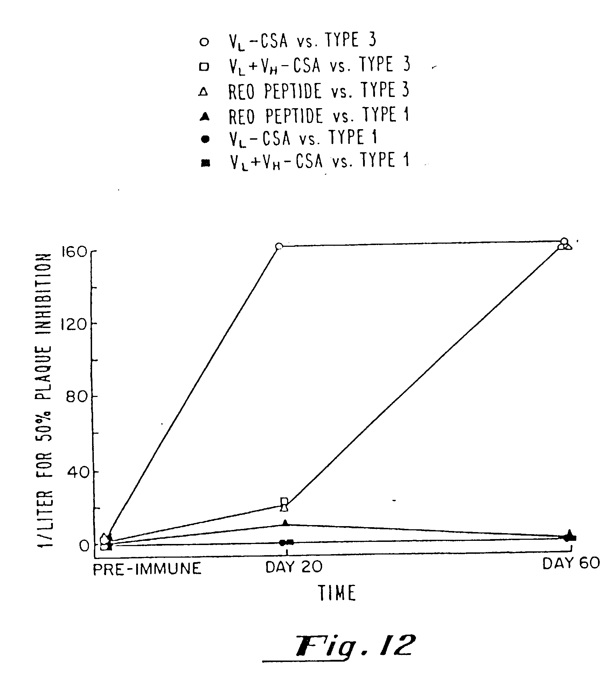

Figures 10 and 11 illustrates plaque inhibition, determined as indicated in the following description; plaque numbers were determined for 4 mice in each group and the mean values determined; the highest dilution of serum that produced 50% or greater plaque inhibition was determined and is shown for each time point at which serum was obtained; plaque inhibition of both type 1 and type 3 virus is shown.

Figure 12 illustrates data for mice immunized with the reovirus types noted by injection of 10⁷ PFU subcutaneously, or with the peptides noted at a dose of 100 ug split into two injections subcutaneously; one week later, mice were challenged with virus or peptides in the footpads; footpad swelling was determined as indicated in the following description 48 hours after challenge; the mean ± SEM for groups of mice is shown.

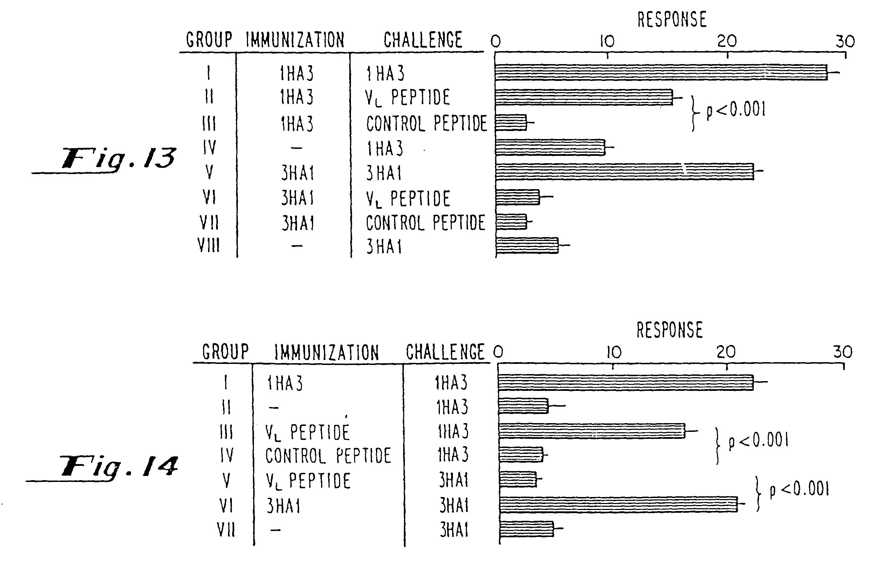

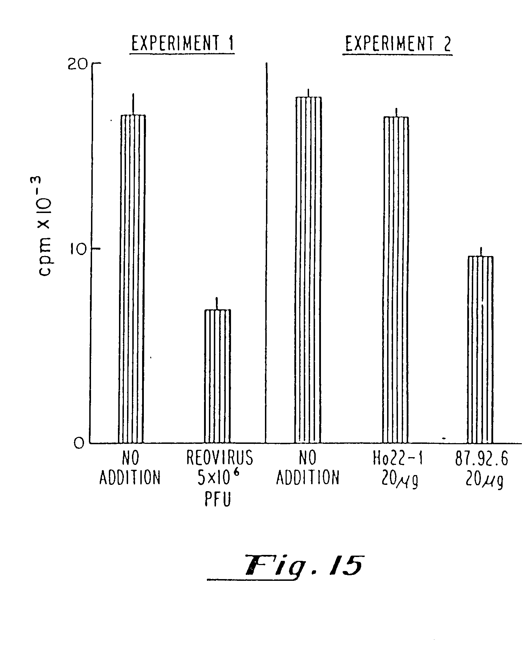

Figures 13 and 14 shows the delayed type hypersensitivity (DTH) response of mice to intact reovirus type 3 after immunization with peptides.

Figure 15 shows reovirus type 3 and 87.92.6 antibody inhibition of L cell proliferation.

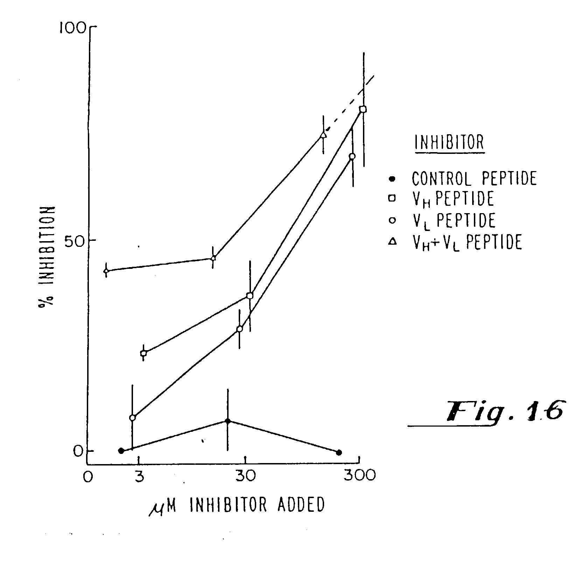

Figure 16 shows competition of binding of 9BG5 antibody to 87.92.6 antibody coated wells in the presence of peptide inhibitors including V_{H}+V_{L} peptide.

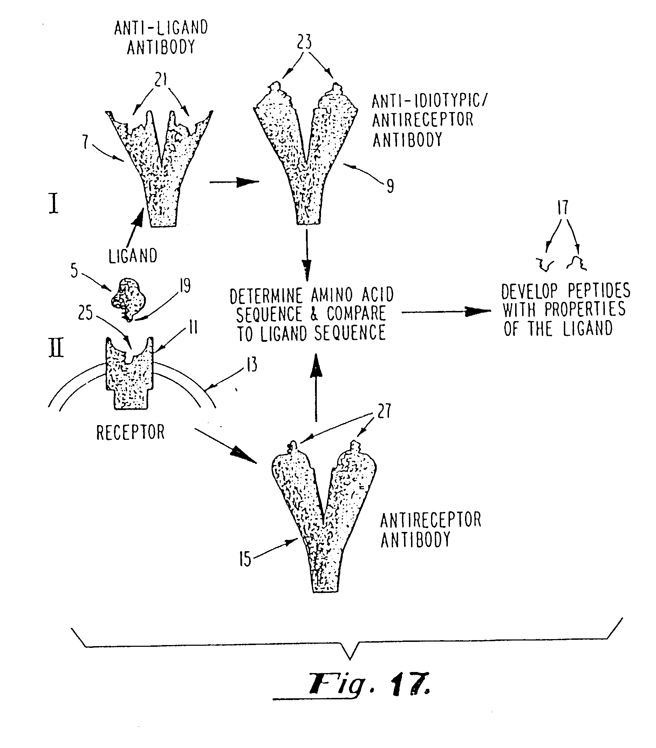

Figure 17 shows a representational diagram of two alternate routes for the development of biologically active peptides according to the methods of the invention.

### DESCRIPTION OF THE PREFERRED EMBODIMENTS

The preferred embodiments of the present invention are described in connection with experiments which have been conducted using reovirus types 1 and 3 interactions with cellular receptors using the anti-idiotype approach. These example(s) were performed using the following experimental procedures, unless otherwise noted.

### Experimental Procedures

### Mice

Adult Balb/c female mice, 6 to 8 weeks to age, were obtained from Jackson Laboratories, Bar Harbor, ME. Pre-immune serum was obtained on all mice used and assayed by neutralization of reovirus infectivity (see below) to ascertain that there had been no prior exposure to reovirus. ice immunized with peptides were housed in the animal care facility and fed a house diet ad libitum (Purina, St. Louis, MO). Mice immunized with reovirus type 3/Dearing were housed in a separate facility.

### Viruses

Reovirus type 1 (Lang), and reovirus type 3 (Dearing) and the reassortants 3.HA-1 and 1.HA-3 have been previously described (Fields, B.N. and Greene, M.I., "Molecular Mechanism of Viral Pathogenesis: Implications for Prevention and Treatment", Nature, 20:19-23, 1982). Clones 1.HA-3 and 3.HA-1 are single segment reassortant clones that segregate the S1 gene, the gene encoding the viral attachment polypeptide (hemagglutinin) sigma 1. For mouse inoculation and virus neutralization, a stock of reovirus that was passed twice in L-cells was purified by substituting ultrasonic disruption (Branson Ultrasonic 200) for cell homogenization in a modification of published techniques (Joklik, W.K., "Studies on the Effect of Chymotrypsin on Reovirions", Virology, 49:700-715, 1972). The number of particles per ml was determined by optical density at 260 nm (Smith, R.E., Zweernik, H.J., and Joklik, *W.K.,* "Polypeptide Components of Virions, top Component and Core of Reovirus Type 3", Virology, 39:791-810, 1969).

### Monoclonal Antibodies

Type 3 reovirus neutralizing monoclonal antibody 9BG5 (mouse IgG2aK) (Burstin, S.J., Spriggs, D.R., and Fields, B.N., "Evidence for Functional Domains on the Reovirus Type 3 Hemagglutinin Virology, 117:146-155, 1982) was purified from hydridoma supernatant with the cells grown in Dulbecco's minimal essential media (DMEM) (MA Bioproducts, Walkersville, MD) with added penicillin/streptomycin solution (The Cell Center, University of Pennsylvania, Philadelphia, PA), and 10% fetal bovine serum (FBS). Culture supernatants were precipitated with 50% (NH₄)₂SO₄, solubilized in distilled water and dialyzed against 3 changes of phosphate buffered saline (PBS). Next the antibody was purified on a Sepharose-protein A column and eluted with 0.1 M citric acid pH 4.5. The eluate was collected in 1 M tris buffer, pH 8.5 to neutralize excess acidity and dialyzed against 3 changes of PBS. The dialysate was concentrated on an Amicon protein concentrator with a molecular weight cut-off of 30 kilodaltons (KD). The purified antibody was more than 95% pure by sodium-dodecyl sulfate polyacrylamide gel electrophoresis (SDS-PAGE). Irrelevant monoclonal antibodies UPC-10 and All (both mouse IgG2aK) was similarly purified from clarified ascites (Gibco, Grand Island Biological Co.).

Monoclonal antibodies 87.92.6 (mouse IgM,K) and HO 13.4 (mouse IgM,K,anti-Thy 1.2) and HO 22.1 (mouse IgM, K, anti Thy 1.1) were purified from 50% ammonium sulfate cuts of culture supernatant or from ascites supernatants from ascites generated in hydridoma bearing Balb/c mice. These preparations were dialyzed against 3 changes of PBS and run over a goat anti-mouse IgM Affigel-10 column. Antibodies were eluted with 3.5 M MgCl₂, dialyzed against 3 changes of PBS and concentrated as noted above. Purity of all monoclonal antibodies used was greater than 95% by SDS-PAGE.

### Cell Lines

Murine L-cells were grown in spinner bottles with Joklik's MEM (MA Bioproducts) with 5% FBS. R1.1 cells (murine thymoma, Thy 1.2+) were grown in suspension in RPMI 1640 (MA Bioproducts, Walkersville, MD) supplemented with L-glutamine, 10 mM HEPES buffer (MA Bioproducts) and penicillin/streptomycin with 10% FBS.

### Immunization of Mice

For the study of DTH response, groups of mice were inoculated with either synthetic peptide or live reovirus type 3 subcutaneously (s.c.) in 2 separate sites on the dorsal flanks of the animal (over each hind limb); 50 ug of a synthetic peptide or 10⁷ viral particles/0.2 ml were given in separate injections of 0.1 ml vol. Six days later, animals were challenged in the left footpad with 3x10⁷ viral particles suspended in saline containing 2% gelatin (30 ul). Footpad swelling was recorded 24 hr later in a blind fashion (Greene, M.I. and Weiner, H.L., "Delayed Hypersensitivity in Mice Infected with Reovirus, II. Induction of Tolerance and Suppressor T Cells to Viral Specific Gene Products", J. Immunol., 125:283-287, 1980). Four animals per group were studied, and the magnitude of the response was determined by comparing the challenged left footpad to the untreated right footpad.

For the study of humoral immune response, mice were inoculated with either synthetic peptide or live reovirus type 3 as above with the following modification. The peptide was conjugated with chicken serum albumin (CSA) as described below and 100 ug of the peptide conjugate was inoculated s.c. in 2 divided doses. For mice immunized with synthetic peptides, the first immunization was with peptide mixed with an equal volume of complete Freund's adjuvant; whereas with subsequent immunization the peptide was suspended in saline containing gelatin. Mice were immunized weekly for five weeks, and serum was obtained prior to the first inoculation, and then at the second and sixth week. For mice immunized with reovirus type 3, 10⁷ plaque forming units (PFU) was inoculated s.c. on the first and third week.

### Radioimmunoassay Procedure

The wells of 96 well V-bottom polystyrene plates (Dynatech Laboratories, Alexandria, VA) were coated with peptide by diluting the peptides to 25 ug/ml in distilled water and evaporating 50 ul in each well by incubating the plates overnight at 37°c. Wells were coated with reovirus type 1 or type 3 by diluting stock solutions of virus to 4.8 x 10¹¹ particles/ml in 0.1 M NaHCO₃ pH 9.5, dispensing 25 ul per well and incubation overnight at 4°C (London, S.D., Rubin, D.H., and Cebra, J.J., "Gut Mucosal Immunization with Reovirus Serotype I/L Stimulates Viral Specific Cytotoxic T Cell Precursors as Well as IgA Memory Cells in Peyer's Patches", 1987). Following overnight incubation, peptide or virus coated wells were washed 3 times with PBS and blocked with 200 ul/well of 1% gelatin in PBS with 0.1% NaN₃ by incubation for 2 hours at 37°C. The wells were decanted, washed 3 times in PBS, and mouse serum or purified monoclonal antibody was added, 50 ul/well, diluted in PBS containing 0.5% gelatin and 0.1% NaN₃. Following a 3 hour incubation at 37°C, the wells were decanted, washed 3 times in PBS, and radioiodinated goat anti-mouse Kappa diluted in PBS 0.1% NaN₃ with 1 mg/ml chicken gamma globulin was added, 100 ul = 48,000 counts per minute (CPM) per well. The plates were incubated overnight at 4°C, decanted, washed ten times in tap water and dried under a heat lamp. Wells were then cut out using a hot wire and counted in a gamma counter. The CPM determined on blank wells not coated with antigen is subtracted from CPM values determined on antigen coated wells in all cases.

### Fluorescence Activated Cell Sorter (FACS) Analysis

R1.1 cells (99% viability to trypan blue dye exclusion) were centrifuged and washed twice in PBS 0.1% NaN₃ with 1% bovine serum albumin (FACS media). Cells were resuspended at 10⁷/ml either in FACS media alone or FACS media containing peptide-BSA conjugates at 200 ug/ml. The cells were incubated on ice for 45 minutes prior to addition of monoclonal antibodies from 0.5 mg/ml stock solutions to 100 ul aliquots to the final concentrations noted. Following an additional 30 minute incubation, 500 ul of FACS medium was added to each sample, the cells were centrifuged, washed once in 500 ul FACS media, resuspended in 10 ul FACS containing a 1:200 dilution of fluoresceinated goat anti-mouse Fab (Southern Biotechnology Associates) and incubated for 30 minutes on ice. 500 ul of FACS media was added, the cells were centrifuged and washed in 500 ul FACS media, resuspended in 200 ul FACS media and analyzed at the University of Pennsylvania fluorescence activated cell sorter.

### Neutralization of Virus Infectivity

The titer of neutralizing antibodies in serum sample were determined in the following manner: (i) Microneutralization: L-cells (5x10⁴ per well) were incubated in 96 well dishes overnight at 37°C. Reovirus type 1/Lang (1/L) and type 3/D were serially diluted and incubated for 1 hour with the L-cells at 37°C. An additional 75 ul of MEN supplemented with 5% fetal bovine serum, 1% glutamine was placed in each well. At 3 days following incubation at 37°, the media containing virus was removed and the cells were stained with Gentian Violet (Gentian Violet 3.4 g/l, ammonium oxalate 8 g/l). The titer of virus used for neutralization was 4 fold in excess of that quantity of virus that was lytic for the L-cell monolayer. Reovirus type 1/L or 3/D at the appropriate concentration was incubated with an equal volume of mouse serum for 1 hr at 25°C om 96 well plates. The virus-serum mixture was then transferred to L-cell monolayers as above. The titer of antibody was determined as the amount which preserved 70% of the monolayer as determined by visual inspection. (ii) Virus plaque reduction: 100 pfu of reovirus type 1/L incubated for 1 hour with L-cells (7x10⁵ cells per well) in 12 well Costar plates. The titer of virus in each well was then determined as previously described (Rubin, D.H., Kornstsein, M.J., and Anderson, A.D., Reovirus Serotype 1 Intestinal Infection: A Novel Replicative Cycle with Ileal Disease", J. Virol., 53:391-398, 1985).

### Synthesis of Peptides:

Peptides were synthesized using a model 430A Applied Biosystems Peptide Synthesizer (Applied Biosystems, Inc., Foster City, CA. Deprotection and release of the peptide from the solid phase support matrix were accomplished by treating the protected peptide on the resin with anhydrous HF containing lot aniscle or 10% thioanisole for 1 to 2 hr at 0 degrees C. The peptides were then extracted with either ethyl acetate or diethylether and then dissolved in 10% aqueous acetic acid and filtered to remove the resin. After lyophilization, the composition and purity of the peptides were determined with both amino acid analysis and reverse phase high performance liquid chromatography. This procedure was used for the synthesis of all peptides, including V_{L} and the variant peptides of V_{L}.

### Conjugation of Peptides to Chicken Serum Albumin (CSA)

Prior to conjugating the peptides to CSA, the CSA was first derivatized with a nucleophilic spacer consisting of adipic dihydrazide, as described by Schneerson, et al., "Preparation, Characterization and Immunogenicity of Haemophilus Influenzae Type b Polysaccharide Protein Conjugates", J. Exe. Med., 152:361, (1980). 30 Mg of the adipic dihydrazide-derivatized-CSA (CSA-ADH) in 5 ml 0,1M sodium bicarbonate was reacted for 15 min at room temperature with 7 mg m-maleimidobenzoylsulfosuccinimide ester (Pierce). To this reaction mixture was then added 50 mg peptide and the couples reaction was allows to proceed at 25 degrees C for 2 hr. Following dialysis against 0.1M ammonium bicarbonate and lyophilization, the CSA-ADH-peptide conjugates were obtained as dry white powders.

### Determination of Shared Peptide Sequence

Prior work has shown that a monoclonal antibody denoted 87.92.6 raised against monoclonal neutralizing anti-reovirus antibody 9BG5 mimics the intact virus by binding to cell-surface receptors specific for type 3 reovirus Noseworthy, J.H., Fields, B.N., Dichter, M.A., Sobotka, C., Pizer, E., Perry, L.L., Nepon, J.T., and Greene, M.I., "Cell Receptors for the Mammalian Reovirus. I. Syngeneic Monoclonal Anti-Idiotypic Antibody Identifies a Cell Surface Receptor for Reovirus", J. Immunol., 131:2533-2538, 1983; Kauffman, R.S., etal, 1983 supra: Co, M.S., Gaulton, G.N., Fields, B.N., and Greene, M.I., "Isolation and Biochemical Characterization of the Mammalian Reovirus Type 3 Cell-Surface Receptor", Proc. Natl. Acad. Sci. USA, 82:1494-1498, 1985. 87.92.6 competes with reovirus type 3 for binding to specific cellular receptors thereby mimicking the viral cell attachment protein sigma 1 (the viral hemagglutinin) in its binding domain. This domain is also implicated in the neutralizing antibody response (Burstin, S.J., et al, 1982 supra; Spriggs, D.R., Kaye, K., and Fields, B.N., "Topological Analysis of the Reovirus Type 3 Hemagglutinin Virology, 127: 220-224, 1983). This implies that 87.92.6 mimics the epitope on the hemagglutinin that interacts with the cellular receptor for reovirus. The nucleic acid sequences of the heavy and light chain variable regions (V_{H} and V_{L} respectively) of 87.92.6 have recently been determined (Bruck, C., Co, M.S., Slaoui, M., Gaulton, G.N., Smith, T., Fields, B.H., Mullins, J.I., and Greene, M.I., "Nucleic Acid Sequence of an Internal Image-Bearing Monoclonal Anti-Idiotype and its Comparison to the Sequence of the External Antigen", Proc. Natl. Acad. Sci. USA, 82:6578-6582, 1986), and the sequences have been compared to that of the reovirus type 3 sigma 1 protein (Bassel-Duby, R., Jayasuriya, A., Chatterjee, D., Sonenberg, N., Maizel, J.V., Jr., and Fields, B.N., "Sequence of Reovirus Hemagglutinin Predicts a Coiled-Coil Structure", Nature, 315:421-423, 1985). In accordance with the methods of the present invention, shared sequence portions of the antigen and anti-idiotype have been identified. More particularly, a 16 amino acid sequence in the reovirus type 3 sigma 1 protein encompassing amino acids 317 and 332 has been identified as having amino acid sequence similarity to a combined sequence encompassing the second complementarity determining regions (CDR IIs) of the 87.92.6 heavy and light chain variable regions (V_{H} and V_{L} respectively). Specifically amino acids 43-51 of the V_{H} share sequence similarity with amino acids 317-324 of sigma 1 and amino acids 46-55 of the V_{L} share homology with amino acids 323-332 of sigma 1 (Bruck, C., et al, 1986, supra).

In accordance with the methods of the present invention peptides corresponding to amino acids 317-332 of the sigma 1 protein 43-50 of the V_{H} sequence and 39-55 of the V_{L} sequence have been synthesized. As demonstrated hereinafter, immunization of Balb/c mice with these peptides results in neutralizing anti-reovirus type 3 antibodies and specific cell-mediated immunity to reovirus. This establishes that the sequence homology between the sigma 1 cell attachment protein and the anti-receptor antibody predicts the neutralizing epitope on the reovirus hemagglutinin, sigma 1. This approach allows the rapid delineation of neutralizing epitopes on pathogens and the development of peptide vaccines that elicit a neutralizing response.

### Binding of Neutralizing Monoclonal Antibody 9BG5 to Peptides

The monoclonal anti-receptor antibody 87.92.6 binds to both the reovirus type 3 receptor and the neutralizing antibody 9BG5 (Kauffman, R.S., et al, 1983, supra). Applicants predicted that the peptides derived from the areas of homology between 87.92.6 and the type 3 reovirus sigma 1 protein (Bruck, C., et al, 1986 supra) would have similar properties. The peptides synthesized to test this hypothesis are shown in Table 1.

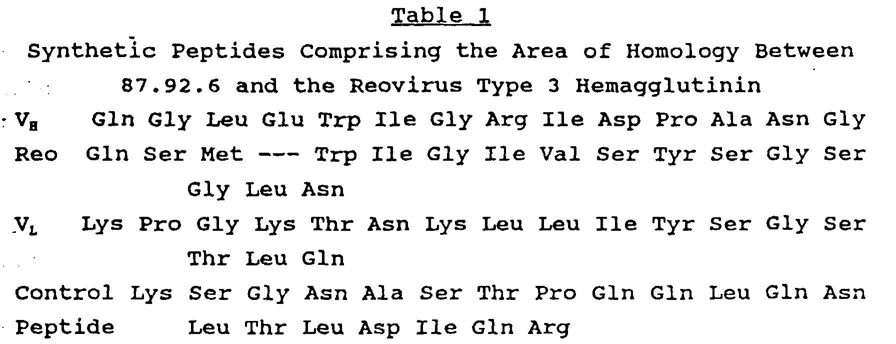

The peptides used in this study were synthesized by solid-phase methods as noted above. The sequences are shown aligned with maximum homology. The amino acids marked with a closed circle are identical and those marked with an open circle are of the same class. It will be noted that the tested peptides contain anti-idiotypic antibody residues in addition to the shared peptide sequence.

The reo peptide corresponds to amino acids 317-332 in the type 3 viral hemagglutinin. Computer modeling predicts this area to be predominantly a beta-sheet configuration and to include a beta-turn. The V_{L} peptide represents amino acids 39-55 of the light chain variable region of 87.92.6, and includes the second complementarity determining region (CDR II). Modeling predicts this area also to be a predominant beta-sheet and to include a beta-turn. The V_{H} peptide comprises amino acids 43-56 of the heavy chain variable region of 87.92.6 including CDR II of the heavy chain. The control peptide, unrelated to this system, is also shown.

Based on these similarities in primary and secondary structures, it was predicted that the reo and V_{L} peptides should be recognized by anti-reovirus type 3 neutralizing monoclonal antibody 9BG5. In Figure 1 we show the results of a radioimmunoassay determining the binding of purified monoclonal antibody 9BG5 to the wells of microtiter plates coated with the peptides. To control for non-specific binding to the polystyrene wells, counts per minute (CPM) determined on blank wells not coated with peptide is subtracted from CPM values determined on peptide coated wells. In addition, since these peptides may also cause non-specific adherence of immunoglobulin molecules, we determined the specific binding of the class-matched irrelevant monoclonal antibody UPC-10 to peptide coated wells and subtracted this value from those determined for 9BG5. No significant binding was seen to the control peptide used in this study. Similarly, binding to the V_{H} peptide only achieved background levels indicating that this epitope is not recognized by 9BG5. There was a small amount of binding to the V_{L} peptide, which has strong homology in its carboxy terminal sequence to the reo peptide carboxy terminal. Although slight, this finding was reproducible on subsequent assays. Strong reproducible binding to the reo peptide by 9BG5 was evident. Since 9BG5 is a neutralizing antibody, this datum implies that the reo peptide contains the neutralizing epitope recognized by 9BG5. The binding to the V_{L} peptide indicates that the area of sequence homology between these peptides (amino acids 323-332 of the sigma 1 protein) is involved in the neutralizing epitope.

### Binding of V_{L} Peptide to the Reovirus Receptor

Prior work indicated that the neutralizing epitope recognized by 9BG5 is involved in binding to the type 3 reovirus receptor (Kauffman, R.S., et al, 1983, supra; Noseworthy, J.H., et al, 1983, supra; Spriggs, D.R., et al, 1983, supra). This led us to speculate that the V_{L} peptide might also interact with the viral receptor. To test this hypothesis we coupled the V_{H} and V_{L} peptides to BSA by incubating peptides and BSA in 0.1% glutaraldehyde followed by dialysis against PBS and used these preparations to determine if we could specifically block 87.92.6 binding to the type 3 reovirus receptor on R1.1 cells. As shown in Figure 2, pre-incubation of R1.1 cells with V_{L}-BSA blocked the binding of 87.92.6 indicating interaction of V_{L}-BSA with the reovirus receptor. This blocking effect is specific as pre-incubation of R1.1 cells with V_{L}-BSA had no effect on the binding of HO 13.4, and isotype matched control monoclonal antibody that binds to the Thy 1.2 molecule on the R1.1 cell surface (Figure 3). These observations were consistently reproducible on multiple experiments. An additional control is shown in Figure 3 where it is demonstrated that V_{H}-BSA has no inhibitory effect on 87.92.6 binding when used at the same concentrations as V_{L}-BSA. These data indicate a direct interaction of the V_{L} peptide with the reovirus type 3 receptor and imply that residues 46-55 of the 87.92.6 V_{L} chain and 323-332 of the type 3 sigma 1 protein directly interact with the reovirus type 3 receptor.

### Immunization with Peptides Induces Reovirus-Binding Antibodies

The V_{L} and reo peptides contain the epitope involved in the interaction between type 3 reovirus and its specific cellular receptor. Immunization of mice with these peptides was done to determine whether they would induce antibodies capable of interacting with reovirus type 3 and blocking infection. Groups of Balb/c mice were immunized with these synthetic peptides as noted in the experimental procedures section. Groups of 4 mice received either control peptide in adjuvant, V_{L} peptide coupled to chicken serum albumin (V_{L}-CSA) in adjuvant, V_{H} and V_{L} peptide coupled to CSA (V_{H} + V_{L} -CSA) in adjuvant, reo peptide in adjuvant or reo peptide without adjuvant. As a positive control an additional group of mice was injected with reovirus type 3. As indicated below, pre-immune serum from these mice disclosed no reovirus neutralizing antibodies indicating no prior exposure to virus.

Radioimmunoassay indicated a strong response to the immunizing antigen in all cases (data not shown). Binding of immune serum (day 60) to reovirus type 1 and type 3 is shown in Figures 4-7. Specific binding was determined by subtracted CPM bound on a blank plate from CPM bound on a virus coated plate. As a further control, specific binding of normal mouse serum to virus coated plates was also subtracted. To simplify interpretation, specific binding is shown for four groups of animals: These immunized with the control peptide, V_{L}-CSA, V_{H}+V_{L}-CSA (all with adjuvant), and reo peptide without adjuvant. Mice immunized with reo peptide plus adjuvant made a response similar to those immunized with V_{L}-VSA or V_{H}+V_{L}-CSA plus adjuvant. Mice immunized with type 3 reovirus made a strong response to type 3 virus (specific CPM at a 10⁻³ dilution of serum of 10,428±807) with significant cross-reactivity with type 1 virus (specific CPM at a 10⁻³ dilution of 6,976±915). As shown in Figures 4-7, serum from mice immunized with control peptide bound poorly to type 1 or type 3 virus coated plates at any of the serum dilutions used. In contrast, significant binding of immune serum to type 1 and type 3 virus coated plates is demonstrated from mice immunized with V_{L}-CSA, V_{H} + SA or reo peptide. As was expected, binding to type 3 virus was significantly higher than binding to type 1 virus, although some cross-reactivity is seen. The binding of type 3 virus was significantly higher than binding to type 1 virus, although some cross-reactivity is seen. The binding of type 1 virus was likely to have been due to some areas of primary sequence homology between the peptides used here and the type 1 sigma la protein (Manemitsu, S.M., Atwater, J.A., and Samuel, C.E., "Biosynthesis of Reovirus-Specified Polypeptides. Molecular cDNA Cloning and Nucleotide Sequence of the Reovirus Serotype 1 Long Strain Bicistronic SlmRNA Which Encodes the Minor Capsid Polypeptide /a and the Nonstructural Polypeptide 16NS", Biochem. Biophys. Res. Commun., 140:501-510, 1986).

These results indicate that primary mice with peptides modeled from the putative neutralizing epitope of type 3 reovirus or the corresponding epitope from the anti-receptor monoclonal antibody induces reovirus binding antibodies.

### Neutralization of Viral Infectivity by Immune Serum from Peptide Immunized Mice

Serum from peptide immune animals was assayed at three time points to evaluate its effects on viral infectivity of L-cells. Two assays were used to detect neutralization of infectivity. One was a direct cytotoxicity assay measuring the effect of serum on viral lysis of L-cells grown adherent to the wells of 96-well microtiter plates by vital staining, and the other was by measuring inhibition of plaque formation by serum, with virus and L-cells in soft agar. Results from the direct cytotoxicity assay are shown in Figures 8 and 9. Pre-immune serum from all of the animals used was assayed and no significant effect on type 1 or type 3 viral lysis of L-cells as demonstrated. As a positive control, neutralization of L-cell lysis by reovirus was demonstrated by serum from mice immunized with reovirus type 3. This serum produced potent inhibition of lysis by both type 3 and type 1 virus, although a preferential effect on type 3 viral lysis was noted, with neutralization titers of 1:512 for type 3 virus on days 20 and 60, and titers of 1:342 and 1:256 for control peptide immunized animals had no effect on L-cell lysis by reovirus type 1 or type 3. Serum from mice immunized with V_{L}-CSA, V_{H} + V_{L}-CSA, or reo peptide with or without adjuvant specifically neutralized L-cell lysis by reovirus type 3 but not type 3 (Figure 8 versus 9). As results were similar for serum from animals immunized with reo peptide in the presence or absence of adjuvant, results only from the latter group is shown. This effect was also seen when serum from these mice was assayed for inhibition of plaque formation. In Figures 10 and 11, the reciprocal serum titer producing 50% of greater plaque inhibition is shown for type 1 and type 3 virus from the groups immunized with V_{L}-CSA, V_{H} + V_{L}-CSA or reopeptide without adjuvant. Again, specific inhibition of plaque formation of type 3 but not type 1 virus is seen. Since peptide-immune serum specifically inhibits type 3 but not type 1 viral infectivity, these peptides define the neutralizing epitope present on reovirus type 3.

### Elicitation of Delayed-Type Hypersensitivity (DTH) to Reovirus Type by Immunization with Peptides

Prior studies have demonstrated that the specificity of DTH responses to reovirus infection involved the sigma 1 polypeptide (Weiss, H.L., Greene, M.I., and Fields, B.N., "Delayed Type Hypersensitivity to Mice Infected with Reovirus. Identification of Host and Viral Gene Products Responsible for the Immune Response", J.Immunol., 125:278-282, (1980). It was therefore determined if immunization of mice with these peptides would elicit DTH responses to intact reovirus. As shown in Figures 13 and 14, significant DTH responses to reovirus type 3 were induced by immunization with V_{L} peptide. This response was type specific as these animals did not demonstrate significant DTH responses to reovirus type 1. Use of reassortant viruses maps the response to the sigma 1 protein. In addition, priming animals with type 3 virus results in significant DTH to the V_{L} peptide. We have also recently demonstrated a type specific proliferative response to reovirus type 3 in spleen cells from mice immunized with reo peptide. These data indicate that V_{L} and reo peptide define an important epitope involved in T cell-mediated immunity to reovirus type 3.

### Binding of Reovirus Type 3 Inhibits Host Cell DNA Synthesis Upon Receptor Perturbation

Reovirus type 3 inhibits host cell DNA synthesis upon receptor perturbation. This effect is not due to infection of cells as replication defective reovirus type 3 particles retain this property. L cells were cultured at 5 X 10⁴ cells per well of 96 well microtiter plates in 100 µl media for 24 hours. Reovirus type 3 particles (A) were added and incubated for an additional 24 hours prior to the addition and incubated for an additional 24 hours prior to the addition of tritiated thymidine. Purified monoclonal antibodies 87.92.6 or HO 22.1 (B) were added for 1 hour at 37°C, then the culture media removed and replaced with 100 µl fresh media for 24 hours, prior to the addition of tritiated thymidine. The cells were incubated for an additional 4-6 hours and counts per minute (CPM) incorporated were determined. Figure 15 shows this effect of reovirus type 3 upon murine fibroblasts. Murine fibroblasts (which possess specific receptors for reovirus type 3) (L cells), were incubated with reovirus type 3, or left untreated (8A). Twenty-four hours later the DNA synthetic level was measured. Reovirus type 3 markedly inhibited DNA syntheses by these cells. 87.92.6 has a similar effect on these cells, as shown in Figure 15. In this experiment, L cells were grown adherent and exposed to antibody for one hour, at which point the antibody was removed, and the cells cultured for an additional 24 hours prior to determination of the DNA synthesis while a control antibody (HO22.1) had no effect. 87.92.6 similarly inhibits DNA synthesis by fibroblasts, neuronal cells and lymphocytes.

### Competitive Binding of 9BG5 to 87.92.6 In The Presence of Peptides

Polystyrene wells were coated with purified 87.92.6 or control IgM, K antibody H)22.1 by incubation of purified antibody (purified on a goat anti-mouse IgM column), diluted in 0.1% NaHCO₃ pH 9.5 to 1µg/ml with 50 µl/well, overnight at 4°C. The wells were washed, blocked with 2% BSA in PBS with 0.1% NaN₃, washed again and mixture of radioiodinated 9BG5 and peptides (at the concentrations noted in Figure 26) were added for one hour at 37°C. The wells were washed and counted. In all cases, specific CPM bound was determined by subtracting CPM bound to blank wells coated with BSA from CPM bound for 87.92.6 coated wells. As shown in Figure 16, binding of ¹²⁵I-9BG5 to wells coated with irrelevant mouse IgM, K antibody HO22.1 was similar to binding to blank wells. Percent inhibition was determined by subtracting specific CPM bound in the presence of inhibitor from specific CPM bound in the absence of inhibitor, dividing this by CPM bound in the absence of inhibitor, and multiplying the result of 100. The ± SEM of values from two experiments is shown in Figure 16.

The present invention approach also proves an alternative route for the development and production of biologically active peptides. As shown in Figure 17, antibodies 15 specific for a receptor 11 of the antigen (or ligand) 5 also mimic the antigen 5 in the same way as an anti-idiotype antibody 9 of the antigen mimics the antigen 5. In pathway I, an antibody 7 contains an epitope designated generally 21 complementary to the neutralizing epitope designated generally 19 of antigen 5. This antibody 7 is then used to produce other antibodies, or anti-idiotype antibodies 9. These anti-idiotype antibodies 9 will have a region designated generally 23 mimicking the neutralizing epitope 19 of the antigen 5. In pathway II, the receptor 11 on cell surface 13 contains an epitope designated generally 25 complementary to the neutralizing epitope 19 of the antigen 5; the antibody specific for the receptor 15 will thus contain a region designated generally 27 mimicking the neutralizing epitope 19 of the antigen 5. The anti-receptor antibody 15 is the equivalent of anti-idiotype antibody 9, since both contain regions (23 and 27) mimicking the neutralizing epitope 19 of the antigen 5. Anti-receptor antibodies 15 can be used as an alternative, or in addition to, anti-idiotype antibodies 9 in the methods described herein to develop and produce biologically active peptides 17 with properties of the antigen or ligand.

Because antigens such as viruses generally contain multiple antigenic epitopes, it may be necessary to screen the antibodies produced in response to the inoculation with the ligand, receptor or anti-ligand antibody to select antibodies having specificity for the neutralizing epitope of the antigen. Screening can be done by competitive assays that determine the antibody's ability to inhibit binding of the antigen to the receptor of the cell, those antibodies having a greater ability to inhibit binding of the antigen containing or mimicking the neutralizing epitope. Suitable screening methods include those described herein, and in It will be obvious to those skilled in the art that various changes to reagents may need to be made in the competitive assays when different antigen and receptor pairs are used.

As demonstrated herein, neutralizing antibody 9BG5, having a specificity for the antigen HA3 on the reo virus, was used to make anti-idiotype antibodies having anti-receptor activity. These anti-idiotype antibodies also bind to the reovirus type 3 receptor. The antibodies were screened to identify antibodies that competed or inhibited binding of the neutralizing antibody with the receptor which would indicate they contained epitopes that mimic HA3, the antigen. The variable region of one antibody having this activity was compared with the sequence of the antigen HA3 to determine regions of homology that define the interaction site of HA3 and the receptor.

Instead of using an anti-receptor antibody that was produced as an anti-idiotype antibody, the receptor itself is also suitable for producing antibodies that have epitopes mimicking the antigen. To produce antibodies by this route, the receptor, typically a protein or glycoprotein is isolated from cells having the receptor by standard techniques for isolating proteins, such as The purified receptor is then used to make antibodies, usually monoclonal antibodies, by conventional techniques. An animal such as a mouse is first injected with the receptor, its spleen cells are removed and fused with myeloma cells to form hybridoma cells, the latter are cloned in a serum-containing medium and the monoclonal antibodies are separated from the medium. The antibodies are then screened by neutralization assay, as described above, to select those antibodies which specifically bind to the receptor site at the neutralizing epitope.

The strategy of utilizing shared primary structure and molecular mimicry to define interacting oligopeptide epitopes thus should have a wide range of applications in the biological sciences to both define areas of specific interaction between molecules, and to aid in the development of substances with predictable biologic activity.

Those of ordinary skill in this art recognize that various modifications can be made in the compounds (peptides) of the present invention without departing from the scope hereof. For example, peptides of the same class (i.e., conservative substitution as described by Chu et al, "Conformational Parameters For Amino Acids in Helical, Beta Sheet, and Random Coil Regions Calculated from Proteins", Biochemistry, 13(2):211 (1974) (which is hereby incorporated by reference) may be substituted in the sequence shared between the antibody and antigen, provided the activity of the resulting peptide is not adversely affected. Additionally, other antibodies, such as other anti-receptor antibodies to the reovirus type 3 receptor or anti-idiotypic antibodies to neutralizing antibodies may also contact the receptor using CDR regions. Peptides derived from these regions having biologic activity similar to that described herein for V_{L} peptide are also within the scope of the invention.

## Claims

1. A method of synthesising biologically active compounds comprising the steps of:
(a) selecting a pathogen product (antigen) known to bind to a physiological receptor of a mammalian cell.
(b) providing an antibody for a cellular receptor of said pathogen gene product;
(c) Determining and comparing the sequences of antigen and the V_{L} and V_{H} regions of the determining region of said antibodies;
(d) synthesizing a peptide comprising an epitope whose sequence matches a homologous region determined as in (c) having at least 60% sequence homology between the V_{L} and V_{H} sequence and the sequence of the antigen,
characterised by the further steps of,
(e) adding cysteine to one end of a peptide of step (d)
(f) adding cysteine to the one end of another peptide of step (d); and
(g) contacting said peptides under conditions selected to allow binding at said cysteine on each peptide to form a composite peptide dimer.

2. A method according to claim 1 wherein said peptide sequences of (e) and (f) are independently (or each) selected from:

X-Lys-Pro-Gly-Lys-Thr-Asn-Lys-Leu-Leu-Ile-Tyr-Ser-Gly-Ser-Thr-Leu-Gln,
X-Lys-Pro-Gly-Lys-Thr-Asn-Lys-Leu-Leu-Ile-Phe-Ser-Gly-Ser-Thr-Leu-Gln,
X-Lys-Pro-Gly-Lys-Thr-Asn-Lys-Leu-Leu-Ile-Tyr-Ala-Gly-Ser-Thr-Leu-Gln,
X-Lys-Pro-Gly-Lys-Thr-Asn-Lys-Leu-Leu-Ile-Tyr-Ser-Ala-Ser-Thr-Leu-Gln, and
X-Lys-Pro-Gly-Lys-Thr-Asn-Lys-Leu-Leu-Ile-Tyr-Ser-Gly-Ser-Ala-Leu-Gln.

3. A method according to either of claims 1 or 2 wherein the antigen is selected from a reovirus gene product.

4. An in vitro method of altering the growth of a mammalian cell comprising administering to that cell a peptide made by a method as claimed in any of claims 1 to 3.

5. A pharmaceutical composition comprising a synthetic biologically active peptide made according to any one of claims 1 to 3 disposed in a pharmacologically acceptable diluent or carrier therefor.

6. A dimer of Claim.

## Patentansprüche

1. verfahren zur Synthese biologisch aktiver Verbindungen, welches folgende Schritte umfasst:
(a) Auswählen eines pathogenen Produkts (Antigen), von dem bekannt ist, daß es an einen physiologischen Rezeptor einer Säugerzelle bindet;
(b) Bereitstellen eines Antikörpers für einen zellulären Rezeptor des pathogenen Genprodukts;
(c) Bestimmen und Vergleichen der Antigensequenzen und der V_{L}- und V_{H}-Regionen der bestimmenden Region des Antikörpers;
(d) Synthese eines Peptids, das ein Epitop umfasst, dessen Sequenz zu einer in (c) bestimmten homologen Region mit mindestens 60 % Sequenzhomologie zwischen der V_{L}- und der v_{H}- Sequenz und der Antigensequenz paßt,
gekennzeichnet durch die folgenden Schritte
(e) der zugabe von Cystein zu einem Ende eines Peptids aus Schritt (d),
(f) Zugabe von Cystein zu einem Ende eines anderen Peptids aus Schritt (d); und
(g) Inkontaktbringen der Peptide unter ausgewählten Bedingungen, die die Bindung des Cysteins an jedes Peptid ermöglichen, um ein zusammengesetztes Peptiddimer zu bilden.

2. Verfahren nach Anspruch 1, worin die Peptidsequenzen von (e) und (f) unabhängig voneinander (oder jeweils) ausgewählt sind aus:

X-Lys-Pro-Gly-Lys-Thr-Asn-Lys-Leu-Leu-Ile-Tyr-Ser-Gly-Ser-Thr-Leu-Gln,
X-Lys-Pro-Gly-Lys-Thr-Asn-Lys-Leu-Leu-Ile-Phe-Ser-Gly-Ser-Thr-Leu-Gln,
X-Lys-Pro-Gly-Lys-Thr-Asn-Lys-Leu-Leu-Ile-Tyr-Ala-Gly-Ser-Thr-Leu-Gln,
X-Lys-Pro-Gly-Lys-Thr-Asn-Lys-Leu-Leu-Ile-Tyr-Ser-Ala-Ser-Thr-Leu-Gln, und
X-Lys-Pro-Gly-Lys-Thr-Asn-Lys-Leu-Leu-Ile-Tyr-Ser-Gly-Ser-Ala-Leu-Gln.

3. Verfahren nach einem der Ansprüche 1 oder 2, worin das Antigen aus einem Reovirus-Genprodukt ausgewählt wird.

4. In-Vitro-verfahren zur Veränderung des Wachstums einer Säugerzelle, das die Verabreichung eines Peptids in diese Zelle umfasst, wobei das Peptid nach einem Verfahren nach einen der Ansprüche 1 bis 3 hergestellt wurde.

5. Eine pharmazeutische Zusammensetzung, die ein synthetisches biologisch aktives Peptid umfasst, das nach einem der Ansprüche 1 bis 3 hergestellt wurde und dafür in einem pharmazeutisch akzeptablen Verdünnungsmittel oder Träger bereitgestellt wird.

6. Ein Dimer nach Anspruch 2.

## Revendications

1. Procédé de synthèse de composés biologiquement actifs comprenant les étapes consistant à :
(a) sélectionner un produit pathogène (antigène) connu pour se lier à un récepteur physiologique d'une cellule de mammifère ;
(b) se procurer un anticorps pour un récepteur cellulaire dudit produit génique pathogène ;
(c) déterminer et comparer les séquences d'antigène et les régions V_{L} et V_{H} de la région déterminante desdits anticorps ;
(d) synthétiser un peptide comprenant un épitope dont la séquence s'apparie à une région homologue déterminée comme dans (c), ayant une homologie de séquence d'au moins 60% entre la séquence V_{L} et V_{H} et la séquence de l'antigène,
caractérisé par les étapes supplémentaires consistant à :
(e) ajouter de la cystéine à l'extrémité d'un peptide de l'étape (d) ;
(f) ajouter une cystéine à une extrémité d'un autre peptide de l'étape (d) ; et
(g) mettre en contact lesdits peptides dans des conditions choisies pour permettre une liaison au niveau de ladite cystéine sur chaque peptide pour former un peptide composite dimère.

2. Procédé selon la revendication 1, dans lequel lesdites séquences peptidiques de (e) et (f) sont choisies indépendamment (ou chacune) parmi :

X-Lys-Pro-Gly-Lys-Thr-Asn-Lys-Leu-Leu-Ile-Tyr-Ser-Gly-Ser-Thr-Leu-Gln,
X-Lys-Pro-Gly-Lys-Thr-Asn-Lys-Leu-Leu-Ile-Phe-Ser-Gly-Ser-Thr-Leu-Gln,
X-Lys-Pro-Gly-Lys-Thr-Asn-Lys-Leu-Leu-Ile-Tyr-Ala-Gly-Ser-Thr-Leu-Gln,
X-Lys-Pro-Gly-Lys-Thr-Asn-Lys-Leu-Leu-Ile-Tyr-Ser-Ala-Ser-Thr-Leu-Gln,
et
X-Lys-Pro-Gly-Lys-Thr-Asn-Lys-Leu-Leu-Ile-Tyr-Ser-Gly-Ser-Ala-Leu-Gln.

3. Procédé selon l'une des revendications 1 ou 2, dans lequel l'antigène est choisi à partir d'un produit génique de Réovirus.

4. Procédé in vitro de modification de la croissance d'une cellule de mammifère comprenant l'administration à cette cellule d'un peptide préparé par un procédé tel que défini à l'une des revendications 1 à 3.

5. composition pharmaceutique comprenant un peptide de synthèse biologiquement actif préparé conformément à l'une quelconque des revendications 1 à 3, disposé dans un diluant ou support pharmaceutiquement acceptable pour celui-ci.

6. Dimère de la revendication 2.
